(19) **Europäisches Patentamt** / **European Patent Office** / **Office européen des brevets**

(11) **EP 4 603 021 A1**

(12) **EUROPEAN PATENT APPLICATION**
published in accordance with Art. 153(4) EPC

(43) Date of publication:
**20.08.2025 Bulletin 2025/34**

(21) Application number: **24845710.3**

(22) Date of filing: **16.01.2024**

(51) International Patent Classification (IPC):
*A61B 5/293* (2021.01)    *A61B 5/263* (2021.01)
*A61B 5/257* (2021.01)    *A61B 5/00* (2006.01)
*A61B 17/00* (2006.01)    *A61B 90/00* (2016.01)
*A61M 5/142* (2006.01)    *A61M 31/00* (2006.01)
*A61N 1/36* (2006.01)

(52) Cooperative Patent Classification (CPC):
**A61B 5/00; A61B 5/257; A61B 5/263; A61B 5/293;
A61B 17/00; A61B 90/00; A61M 5/142;
A61M 31/00; A61N 1/36**

(86) International application number:
**PCT/KR2024/000733**

(87) International publication number:
**WO 2025/023406 (30.01.2025 Gazette 2025/05)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **24.07.2023 KR 20230096129
31.07.2023 KR 20230099677**

(71) Applicant: **Seoul National University R&DB
Foundation
Seoul 08826 (KR)**

(72) Inventors:
• **KANG, Seung Kyun**
  **Seoul 08826 (KR)**
• **BAE, Jae Young**
  **Seoul 08826 (KR)**
• **KIM, Young Seo**
  **Seoul 08826 (KR)**

(74) Representative: **TRBL Intellectual Property
Plaza de Castilla 3, 7°A
28046 Madrid (ES)**

(54) **ELECTRONIC UMBRELLA ELEMENT FOR MINIMALLY INVASIVE SURGERY AND MANUFACTURING METHOD THEREFOR**

(57) The present invention relates to a minimally invasive electronic device for implantation in a living body, aiming to detect signals within the body, stimulate the body, or release drugs through various methods. Through interaction between the internal and external environments of the living body, the invention is intended for use in various purposes such as diagnosis, treatment, and research.

**Fig. 1**

EP 4 603 021 A1

**Description**

**TECHNICAL FIELD**

The present invention relates to an electronic device and a method of manufacturing the same, and more particularly, to an implantable electronic device that can be used for diagnosis, treatment, and research purposes, and a manufacturing method for producing such a device.

**BACKGROUND ART**

Unless otherwise indicated in this specification, the content described in this section is not prior art to the claims of this application, and inclusion in this section does not constitute an acknowledgment that it is prior art. Generally, electronic devices used for medical purposes have the disadvantage of being exposed to various side effects during the process of incising and recovering the skin, as they are implanted inside the body through surgery.

Therefore, there is a need for technology that can reduce the volume of electronic devices during the implantation process to enable implantation of electronic devices through minimally invasive surgery during the process of incising the skin and implanting the electronic device.

In this regard, Korean Registered Patent Publication No. 10-1864033 discloses an invasive bioelectronic device, and Korean Registered Patent Publication No. 10-1744395 discloses a manufacturing method for a transparent substrate for biodegradable flexible electronic devices containing starch.

However, existing inventions do not disclose technology that can minimize body incision by reducing volume through shape transformation during the implantation process.

Additionally, existing foreign disclosed technologies merely reveal some concepts about how expansion can occur inside the body, but there is no technology that specifically addresses which parts of the body can be entered in what form, or how interaction with the target object can occur after entry.

Naturally, biocompatible implantable electronic devices, for which safety verification is extremely important, have hardly any cases that have been tested through actual in vivo experiments. As a result, work toward the commercialization of minimally invasive bioinjectable electronic devices has been progressing slowly.

**TECHNICAL PROBLEM**

The purpose of this invention is to propose a new type of implantable electronic device for minimally invasive procedures that can be injected into the environment between the inner wall of the skull and the brain surface, and utilized for various purposes such as diagnosis, treatment, and research.

The objective of this invention is to provide an electronic device that can be injected into the body using minimally invasive methods in a volume-reduced state through shape transformation, and which automatically transforms its shape under specific conditions in the targeted environment.

In this process, we aim to propose materials and structures for an electronic device that will not sustain damage to its form or structure while being folded or crumpled inside narrow cross-sectional injection instruments (for example, syringes) introduced for minimally invasive surgery, and which can gently unfold and settle without causing damage to the targeted body part when expelled.

Additionally, the present invention aims to enable the device to autonomously change its shape upon implantation in the body, allowing it to unfold and attach over a wide area without requiring the introduction of an external signal. It is also designed to interact with a part of the body by collecting information from the body or applying stimuli.

Another objective of this electronic device is to biodegrade after fulfilling its intended function, ensuring that it completely decomposes within the body and is safely excreted without residue.

The technical challenges addressed by the present invention are not limited to those described above, and it is evident that additional technical objectives may be derived from the following description.

**TECHNICAL SOLUTION**

An embodiment according to one aspect of the present invention, proposed to solve the aforementioned problem, relates to an electronic umbrella device for minimally invasive surgery.

In one embodiment of the present invention, the proposed bio-implantable electronic device comprises: a central support portion; and a plurality of branch portions extending from the central support portion; wherein, when inserted into the environment between the inner wall of the skull and the brain surface, the branch portions unfold, come into contact with the brain surface, operate, and then biodegrade to be excreted from the body.

According to one embodiment, the branch portions include: an electrode; and a cover layer covering the electrode from above and below; wherein the cover layer has an open structure, including a portion where the electrode is exposed externally.

According to one embodiment, the branch portion may include a shape-memory polymer that restores its shape when environmental conditions are satisfied.

According to one embodiment, the environmental conditions may include one or more conditions selected from the group consisting of temperature, humidity, pH concentration, light intensity, ultrasound, and electromagnetic fields of the surrounding environment.

According to one embodiment, the branch portion may include a highly elastic, super-flexible polymer having an elastic range of 250% or more based on Young's modulus and a Young's modulus of 20 kPa or more.

According to one embodiment, the branch portion may further include DNA bonded to the polymer, wherein the DNA may include a hairpin structure or double-stranded DNA.

According to one embodiment, the branch portion may be formed from a material that includes DNA only in certain regions where the mechanism of shape transformation needs to occur.

According to one embodiment, the branch portion may form a network structure.

According to one embodiment, the network structure may include a plurality of chassis portions radially extending from the central support portion; and support portions formed to interconnect the chassis portions.

According to one embodiment, at least a portion of the area where the chassis portion and the chassis support portion are connected may include a device portion.

According to one embodiment, at least a portion of the distal end and the intermediate point of the chassis portion may include respective device portions.

According to one embodiment, the device portion may include at least one selected from the group consisting of a sensor unit, an electrode unit, an electrical stimulation unit, a drug injection unit, an ultrasonic transmission unit, a battery unit, and a light transmission unit.

According to one embodiment, the device portion may be configured such that a single device portion performs multiple functions.

According to one embodiment, at least two of the device portions may perform different functions.

According to one embodiment, the chassis portions having device portions that perform different functions may be assembled to be connected to the central support portion.

According to one embodiment, when the bio-implantable electronic device is in a bundled state and is inserted in a direction where the branch portion first contacts the brain surface, the contact angle at which the distal end of the chassis portion first contacts the target object may be 70 degrees or less.

According to one embodiment, the bio-implantable electronic device may include an X-ray marker.

According to one embodiment, the central support portion may form a pillar-shaped shaft structure in the z-axis direction, and the central support portion may be provided with a means for enabling wireless communication with the outside or may allow a conductive line for wired communication to pass through.

According to one embodiment, the central support portion may include a solid support that supports the standing structure of the central support portion.

In another embodiment proposed by the present invention, the electronic umbrella device for minimally invasive surgery is a bio-implantable electronic device comprising a central support portion; and a branch portion including a plurality of chassis portions extending from the central support portion; and after being inserted into the body while being bundled into a three-dimensional structure in the z-axis direction with a height of 5 mm or less outside the body, it unfolds into a two-dimensional structure in the x-y axis direction inside the body, making minimally invasive surgery possible.

According to one embodiment, the electronic umbrella device may have a ratio of the unfolded area to the folded area of 10 times or more.

In another embodiment proposed by the present invention, the electronic umbrella device for minimally invasive surgery is a bio-implantable electronic device comprising a central support portion; a branch portion including a plurality of chassis portions extending from the central support portion; and a plurality of chassis support portions formed to interconnect the plurality of chassis portions. When inserted into the environment between the inner wall of the skull and the brain surface, the branch portion unfolds, comes into contact with the brain surface, and operates. When the branch portion is inserted in a direction where it first contacts the brain surface, the structure may be such that, upon contact with the brain surface, the uppermost support portion, which is the support portion closest to the central support portion, experiences the greatest amount of strain.

In another embodiment proposed by the present invention, the electronic umbrella device for minimally invasive surgery is a bio-implantable electronic device comprising a central support portion; and a branch portion including a plurality of chassis portions extending from the central support portion; and when inserted into the environment between the inner wall of the skull and the brain surface, the branch portion unfolds, comes into contact with the brain surface, and operates, wherein the branch portion is formed by stacking multiple layers and then being encapsulated and may include at least three layers: a lower cover layer, an electrode, and an upper cover layer.

According to one embodiment, the branch portion may further include at least one additional layer selected from the group consisting of an active layer, an inter-layer, a receiver coil layer, and a gate oxide layer.

Additionally, another embodiment proposed in another aspect of the present invention relates to a method for manufacturing an electronic umbrella device for minimally invasive surgery.

In one embodiment of the present invention, a method for manufacturing an implantable bioelectronic device is proposed, wherein the implantable bioelectronic device operates by making contact with the brain surface as the branched portion unfolds upon entry into the environment between the inner wall of the skull and the brain surface, and comprises: a central support portion; a branched portion provided with a plurality of chassis portions extending from the central support portion; and a step of assembling a plurality of prepared chassis portions to the central support portion.

In another embodiment of the present invention, a method for manufacturing an implantable bioelectronic device is proposed, wherein the implantable bioelectronic device operates by making contact with the brain surface as the branched portion unfolds upon entry into the environment between the inner wall of the skull and the brain surface, and comprises: a central support portion; and a branched portion provided with a plurality of chassis portions extending from the central support portion. The chassis portion may be manufactured by: preparing a lower substrate; forming a lower cover layer on the lower substrate; forming the central support portion on the lower cover layer; forming a laminated structure of a prepared electrode and an upper cover layer on the lower cover layer; and folding up one side of the lower cover layer, the electrode, and the upper cover layer based on the central support portion.

In another embodiment of the present invention, a method for manufacturing an implantable bioelectronic device is proposed, wherein, in the manufacturing method of the implantable bioelectronic device, the implantable bioelectronic device operates by making contact with the brain surface as the branched portion unfolds upon entry into the environment between the inner wall of the skull and the brain surface, and comprises: a central support portion; and a branched portion provided with a plurality of chassis portions extending from the central support portion, wherein the branched portion is manufactured by forming a laminated structure including the lower cover layer, the electrode, and the upper cover layer, and the process of forming the laminated structure includes a step of designing the structure of each layer through a laser process.

An embodiment according to another aspect of the present invention, which is proposed to solve the above-described problem, relates to a method for implanting an electronic umbrella device for minimally invasive procedures.

The method for implanting an implantable bioelectronic device proposed in one embodiment of the present invention relates to a method for implanting an implantable bioelectronic device that, upon entering the environment between the inner wall of the skull and the brain surface, unfolds its branched portion and operates by making contact with the brain surface, wherein the implantable bioelectronic device comprises: a central support portion; and a branched portion including a plurality of chassis portions extending from the central support portion, and includes: a step of folding and placing the implantable bioelectronic device into an implantation device; a step of implanting the implantable bioelectronic device through a hole drilled in the skull; a step in which the implantable bioelectronic device unfolds inside the skull and settles on the brain surface; and a step in which the implantable bioelectronic device operates on the brain surface.

According to one embodiment, the step in which the implantable bioelectronic device unfolds inside the skull and settles on the brain surface may include a step in which the branched portion of the implantable bioelectronic device autonomously unfolds in the environment inside the skull.

According to one embodiment, the step of folding and placing the implantable bioelectronic device into a sealed implantation device that includes a narrow enclosed space may further include a step of injecting a lubricant into the sealed implantation device together with the implantable bioelectronic device, wherein the lubricant may allow the implantable bioelectronic device to smoothly unfold and glide onto the brain surface as it expands and settles on the brain surface.

According to one embodiment, the step in which the implantable bioelectronic device unfolds and settles on the brain surface may allow the implantable bioelectronic device to adhere well to a targeted point on the brain surface by means of an adhesive.

## ADVANTAGEOUS EFFECTS

According to one embodiment disclosed in the present specification, the electronic device has the advantage of providing an implantable electronic device that enables minimally invasive surgery or procedures within the skull.

By using the electronic device proposed in the embodiment of the present invention, it is possible to replace conventional attempts that inevitably involved sensing brain signals or applying stimulation to the brain from outside the skull due to the risk associated with large-area skull incision. Instead, new types of medical procedures, surgeries, and diagnostics can be attempted with only a minimal skull incision.

In addition, when using the electronic device proposed in the present invention, along with its manufacturing and implantation methods, the electronic device can be folded and inserted without issue into the sealed space inside an implantation tool, even when the tool has a very narrow cross-sectional area. Furthermore, as the device exits from the interior of the implantation tool into an external open environment, it unfolds back into its intended shape without malfunction or deformation. Through this process, direct contact with the inner side of the skull using a minimally invasive approach becomes possible, thereby enabling direct interaction with the brain surface.

Furthermore, the effects of the present invention as described herein are naturally exhibited based on the disclosed configuration, regardless of whether they are recognized by the inventor. Therefore, the aforementioned effects represent only some of the effects based on the disclosed content, and should not be construed as an exhaustive enumeration of all effects recognized or realized by the inventor.

Furthermore, the effects of the present invention should be additionally understood based on the overall disclosure of the specification, and even if not explicitly stated in a specific sentence, any effect that a person skilled in the art can recognize from this specification as being present should be regarded as an effect disclosed in this specification.

**BRIEF DESCRIPTION OF THE DRAWINGS**

FIG. 1 is a schematic diagram illustrating a process in which an electronic umbrella device, according to one embodiment of the present invention, is injected in a folded or crumpled state into the inside of the skull through a syringe, unfolds to perform its intended function, and subsequently biodegrades.

FIG. 2 is a schematic diagram illustrating a technical problem in which, when the electronic umbrella device is folded or crumpled and inserted into a narrow implantation device, plastic deformation occurs inside the implantation device, or the device is damaged due to friction with the inner wall when entering or exiting the implantation device, thereby failing to restore its shape as originally designed.

FIG. 3 is a schematic diagram showing the narrow space between the inner wall of the skull and the brain surface, including a photograph and an illustration, representing spatial constraints in which the electronic umbrella device, inserted in a folded or crumpled state into a space with a height of less than 5 mm, must effectively unfold.

FIG. 4 is a schematic diagram illustrating a structure in which the electronic umbrella device, according to one embodiment of the present invention, is initially deployed in a two-dimensional (x-y plane) configuration before implantation, then transitions into a three-dimensional (z plane) structure upon entering the narrow space of the implantation device for minimally invasive insertion, and subsequently reverts to a two-dimensional configuration inside the skull.

FIG. 5 is an image illustrating an example in which different sensors and electrodes are formed on the terminal portion of the branched section of the electronic umbrella device according to one embodiment of the present invention.

FIG. 6 is a schematic diagram showing that different electronic modules can be formed at the terminal portion of the branched section of the electronic umbrella device according to one embodiment of the present invention.

FIG. 7 is a schematic diagram illustrating the laminated structure of the electronic umbrella device according to one embodiment of the present invention.

FIG. 8 includes a schematic diagram (upper figure) showing the central support portion of the electronic umbrella device and an auxiliary support structure (solid support) that supports the implantation structure of the central support portion, and a cross-sectional view (lower figure) illustrating the connection structure between the branched portion and the central support portion of the electronic umbrella device.

FIG. 9 is an image illustrating a structure in which the electrode of the electronic umbrella device, according to one embodiment of the present invention, is wired through the folded central support portion of FIG. 8 and connected to an external electronic device.

FIG. 10 is an image showing the actual fabricated form and scale of the electronic umbrella device according to one embodiment of the present invention, where the laminated structure of the terminal portion of the branched section responds to temperature and unfolds into its original shape.

FIG. 11 is a schematic diagram illustrating the laminated structure when the electronic umbrella device, according to one embodiment of the present invention, is equipped with a wireless communication function.

FIG. 12 is an image showing the fabricated embodiment of the electronic umbrella device, according to one embodiment of the present invention, including an X-ray marker, along with the captured X-ray image.

FIG. 13 is an FEA simulation image illustrating the potential risk of component failure due to excessive strain caused by deformation when the electronic umbrella device, according to one embodiment of the present invention, is initially inserted into the implantation device, along with an actual captured image of a fractured electronic umbrella device with formed cracks.

FIG. 14 is an FEA simulation image showing the planned unfolding of the electronic umbrella device as it is discharged from the implantation device according to one embodiment of the present invention, along with an actual captured image of a failed case where the device did not unfold as intended.

FIG. 15 is the result of a Young's modulus experiment testing the conditions of polymer materials to ensure effective unfolding of the electronic umbrella device in a constrained environment according to one embodiment of the present invention.

FIG. 16 is a simulation image illustrating the results of exploring the optimal structure of the chassis portion and support portion to enable effective unfolding of the electronic umbrella device in a constrained environment as it is discharged from the implantation device according to one embodiment of the present invention.

FIG. 17 is an image showing the design of each configuration of the electronic umbrella device and the corresponding filling ratio values according to one embodiment of the present invention.

FIG. 18 is a schematic diagram illustrating the cross-sectional structure of the lower and upper cover layers around the electrode in the branched portion of the electronic umbrella device according to one embodiment of the present invention.

FIG. 19 is a simulation result analyzing the factors affecting the effective unfolding of the electronic umbrella device when transitioning from a crumpled or folded 3D structure to a 2D structure according to one embodiment of the present invention.

FIG. 20 is an image illustrating the mechanical changes and issues that arise when considering friction on the brain surface in the electronic umbrella device according to one embodiment of the present invention, particularly regarding the reduction (compensation) of the contact angle (chassis angle) at the terminal portion of the chassis.

FIG. 21 is a schematic diagram explaining that the electronic umbrella device according to one embodiment of the present invention can unfold more smoothly when a lubricant is present.

FIG. 22 is an image showing the result of the electronic umbrella device undergoing biodegradation as pre-designed after an appropriate period has elapsed according to one embodiment of the present invention.

FIG. 23 is a process diagram illustrating the manufacturing process of the chassis portion of the electronic umbrella device according to one embodiment of the present invention, showing the cross-sectional structure at each step.

FIG. 24 is a process diagram illustrating the steps of attaching the chassis portion of the electronic umbrella device to the lower substrate and connecting it to the central support portion through cross-sectional structures at each stage according to one embodiment of the present invention. It can be observed that the chassis portion is attached based on the central support portion and folded into an "L" shape, extending in the vertical direction (z-axis direction).

FIGS. 25 to 27 are images and graphs showing the in vivo (in-vivo) experimental results of the electronic umbrella device according to one embodiment of the present invention.

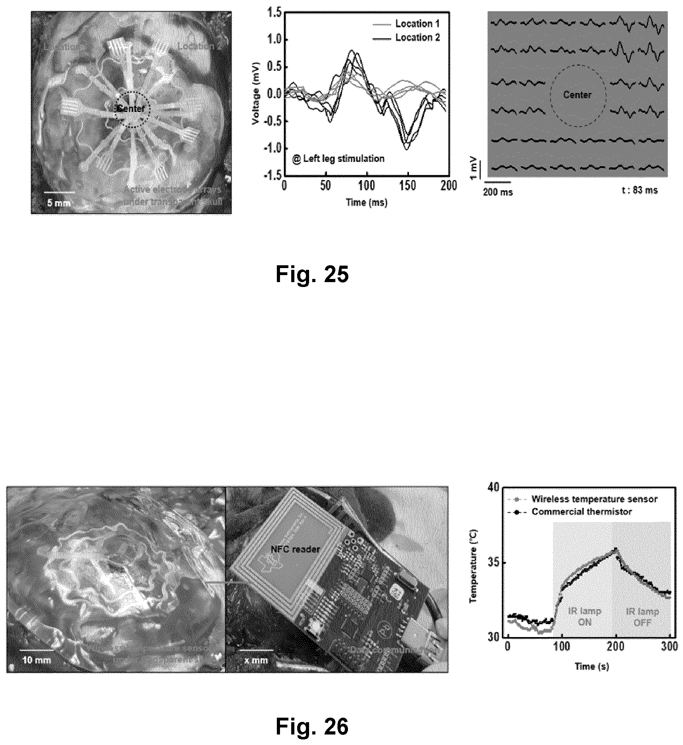

FIG. 25 is an experimental image and graph showing the measured signals transmitted from each component of the electronic umbrella device when the components are attached to different locations on the brain surface of an animal subject according to one embodiment of the present invention.

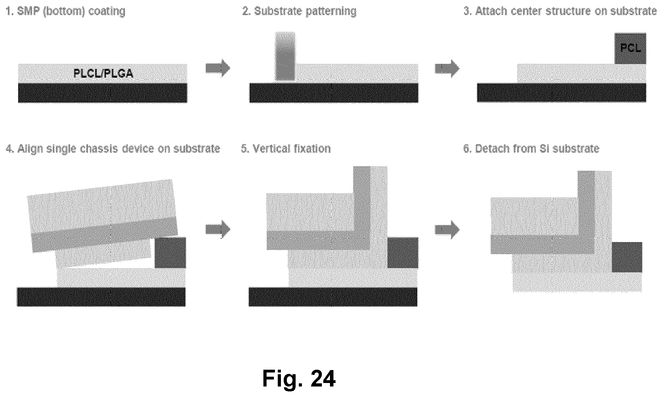

FIG. 26 is a graph illustrating the received temperature signals when the electronic umbrella device, manufactured to enable wireless communication via NFC, is implanted onto the brain surface of an animal subject, and the IR lamp is turned on and off to control temperature according to one embodiment of the present invention. FIG. 24 shows that there is no significant difference between the results obtained using a commercially available temperature sensor in a control experiment and those obtained using the electronic umbrella device of the present invention.

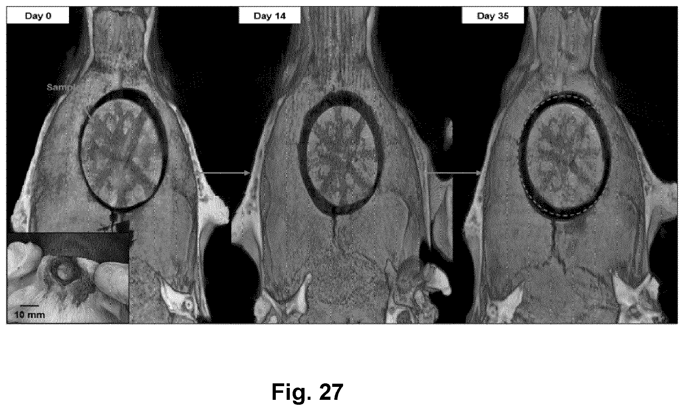

FIG. 27 is an image capturing the process of gradual biodegradation of the electronic umbrella device over 35 days after implantation on the surface of an animal subject according to one embodiment of the present invention. The images in FIG. 27 clearly demonstrate that the biodegradation progresses according to the intended timeframe.

## MODE FOR INVENTION

The embodiments of the present invention are provided as examples for the purpose of explaining the technical concept of the invention. The scope of rights under the present invention is not limited to the embodiments presented below or the detailed descriptions of these embodiments.

All technical and scientific terms used in the present invention shall have meanings generally understood by those skilled in the technical field to which the present invention pertains, unless otherwise defined. All terms used in the present invention are selected for the purpose of providing a clearer explanation of the invention and are not intended to limit the scope of rights under the present invention.

Expressions such as "comprising," "including," and "having" used in the present invention shall be understood as open-ended terms that imply the possibility of including other embodiments unless otherwise specified in the phrase or sentence in which they appear.

Singular expressions described in the present invention shall be construed to include plural meanings unless otherwise specified, and this applies equally to singular expressions stated in the claims.

In the present invention, the height direction of the electronic umbrella device is defined as the vertical direction (z-axis direction), the x-axis direction is defined as an arbitrary direction perpendicular to the vertical direction (z-axis direction), and the y-axis direction is defined as a direction perpendicular to both the x-axis and z-axis directions. Through this definition, the x-y plane becomes a plane perpendicular to the z-axis, and the branched portion of the electronic umbrella device, which will be described later, aims to unfold along the x-y plane in a 2D structure.

Hereinafter, embodiments of the present invention will be described with reference to the accompanying drawings. Additionally, in the description of the following embodiments, repeated descriptions of identical or corresponding components may be omitted. However, even if the description of a component is omitted, it should not be interpreted as an intention to exclude such a component from a particular embodiment.

Generally, implantable bioelectronic devices used for medical applications are inserted into the body through surgical procedures, requiring an incision and subsequent healing of the skin. This process exposes the patient to various complications.

Therefore, to enable the implantation of implantable bioelectronic devices through a minimally invasive approach, a technology that reduces the volume of the device during implantation is necessary.

The implantable bioelectronic device intended in the present invention is designed to be in a folded or crumpled state due to physical and chemical environmental conditions in an external environment or inside an implantation device. When inserted into the body and exposed to specific conditions, it undergoes unfolding or expansion, allowing implantation with minimal incision.

Although the electronic umbrella device proposed in the present invention can be applied to various general-purpose applications, this invention specifically proposes an implantable bioelectronic device designed to be injected and deployed in the environment between the inner wall of the skull and the brain surface.

During the implantation stage, the implantable bioelectronic device is in a relatively compact folded or rolled state compared to its original large-area form, allowing it to be injected into the body through an implantation device (such as a syringe). Once inside the body, during the operational phase, the relatively compacted implantable bioelectronic device restores to its original shape.

The implantable bioelectronic device biodegrades after a predetermined period inside the body. The biodegraded electronic components do not cause any adverse effects on the body and are excreted together with bodily fluids, providing an additional advantage.

The implantable bioelectronic device can be implanted in environments such as between the inner skull and the brain surface, the epidural, or the subdural space, allowing precise measurement of brain signals. Additionally, it enables direct electrical stimulation of the brain surface or drug delivery-based treatment.

Due to the risks associated with skull incision, conventional electronic devices have applied electrical signals externally to the skull, resulting in limited effectiveness in transmitting signals to the brain. However, by using the electronic umbrella device of the present invention, this issue can be resolved without the need for major surgical procedures.

## CONCEPT AND STRUCTURE OF THE ELECTRONIC UMBRELLA DEVICE

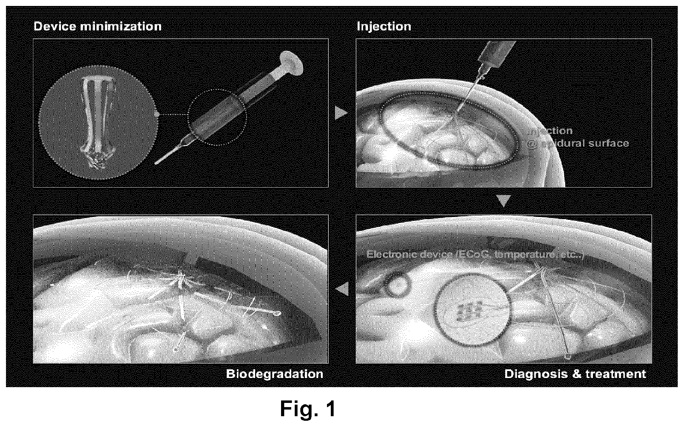

With reference to FIGS. 1 to 22, the concept and structure of the electronic umbrella device proposed in the embodiments of the present invention are described.

FIG. 1 is a schematic diagram illustrating the process in which the electronic umbrella device, according to one embodiment of the present invention, is injected in a folded or crumpled state into the inside of the skull through a syringe, unfolds to perform its intended function, and subsequently biodegrades.

FIG. 1 also illustrates the objective of the inventors in proposing the electronic umbrella device in this embodiment. The electronic umbrella device is first folded and inserted into an implantation device, then injected through a minimally invasive hole in the skull into the epidural surface environment on the brain surface. The electronic device then performs its intended diagnostic and therapeutic functions before biodegrading.

To achieve these technical objectives, the electronic umbrella device must be designed to be effectively folded and inserted into the narrow cross-section of the implantation device, smoothly discharged without failure, and properly unfolded into its original shape after discharge so that it can function before biodegrading.

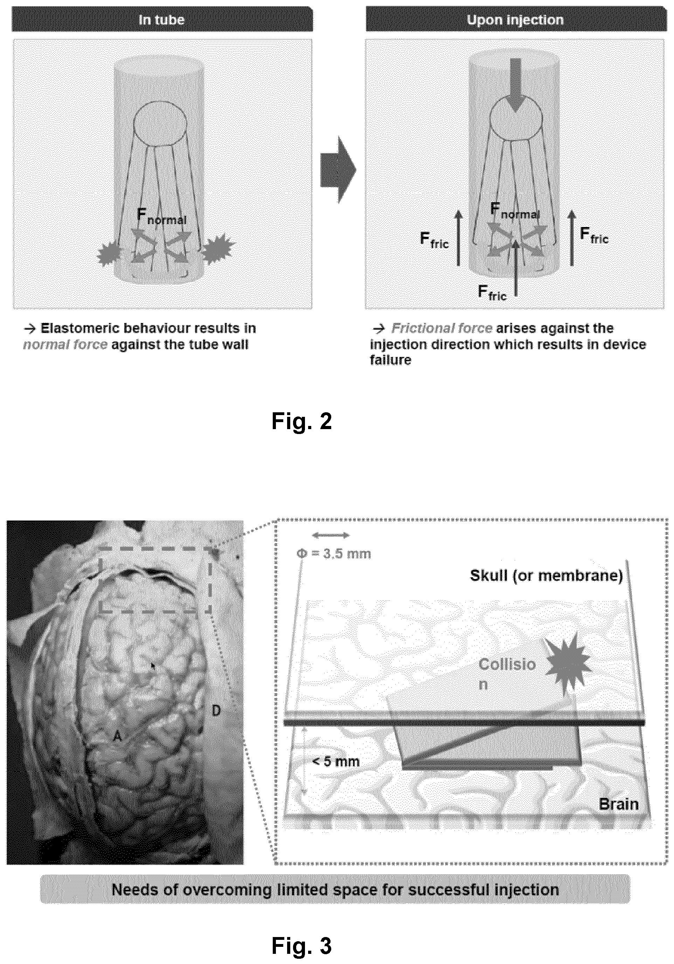

FIG. 2 is a schematic diagram illustrating a technical challenge that arises when the electronic umbrella device is inserted into a narrow implantation device in a folded or crumpled state. Issues such as plastic deformation inside the implantation device or damage due to friction with the inner wall during insertion or removal can prevent the device from restoring its pre-designed shape as intended.

To enable minimally invasive implantation as depicted in FIG. 2, it is crucial to prevent the electronic umbrella device from being tangled, twisted, or stuck due to friction within the narrow space of the implantation device. Additionally, damage to the device caused by friction with the inner wall of the implantation device must be avoided.

Another major technical challenge in ensuring the success of this technology is the extremely confined space inside the skull. In the embodiments of the present invention, the device is designed to be effectively deployed after injection into a narrow space with a thickness of approximately 5 mm.

FIG. 3 is a schematic diagram, including photographs and illustrations, showing the narrow space between the inner skull wall and the brain surface. It represents the spatial constraints in which the electronic umbrella device, inserted in a folded or crumpled state into a space with a height of less than 5 mm, must effectively unfold.

Due to the confined internal body environment, restoring the shape of large-motion electronic devices in a broad planar manner can be challenging. Therefore, the minimally invasive electronic umbrella device intended in the present invention must be capable of restoring its shape even in restricted and narrow environments.

The inventors have recognized these technical objectives and studied various enabling technologies to ensure that an electronic umbrella device, initially deployed in a two-dimensional (2D) configuration, can be compressed inside the implantation device and later redeployed into its original 2D form.

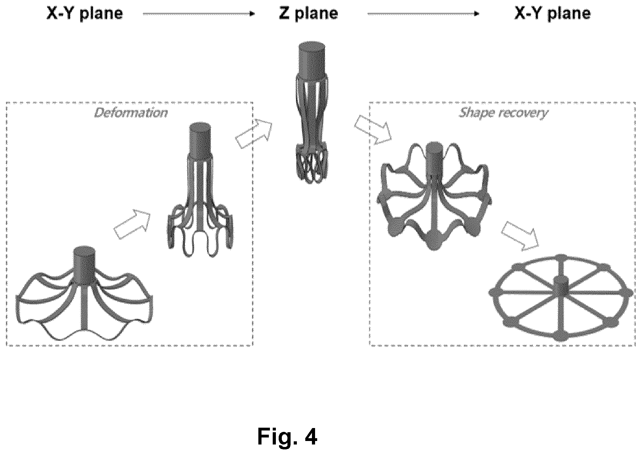

FIG. 4 is a schematic diagram illustrating the structure in which the electronic umbrella device, according to one embodiment of the present invention, is initially deployed in a two-dimensional (x-y plane) configuration before implantation, then transitions into a three-dimensional (z plane) structure upon entering the narrow space of the implantation device for minimally invasive insertion, and subsequently reverts to a two-dimensional configuration inside the skull.

Due to the characteristics of the minimally invasive implantation instrument, the electronic umbrella device is introduced into the body through an insertion tube, which has a small diameter but fewer length constraints. At this stage, the electronic umbrella device is elongated along the z-axis to fit inside the insertion tube.

After being implanted into the body, the electronic umbrella device, which was initially folded along the z-axis, expands into the x-y plane while maintaining a limited height along the z-axis to conform to the constrained internal body environment. This transformation (axis transformation) enables broad-area shape restoration, allowing the device to function effectively in its deployed state.

The following provides a detailed explanation of the structure and materials of the electronic umbrella device to enable the above-described implementation.

In one embodiment of the present invention, the implantable bioelectronic device comprises a central support portion and a branched portion extending from the central support portion. When the implantable bioelectronic device enters the environment between the inner skull wall and the brain surface, the branched portion unfolds and makes contact with the brain surface to operate before biodegrading and being excreted from the body. The branched portion may be configured to include multiple chassis portions.

The central support portion may be designed as a pillar-like structure rising along the z-axis based on a two-dimensional (x-y plane) reference. The central support portion may be designed to accommodate a wired or wireless communication unit. If a wired communication unit is incorporated, the central support portion may include an internal space that allows signal wires to pass through.

In one embodiment, the branched portion may include an electrode and a cover layer that encloses the electrode from above and below. The cover layer may include an upper cover layer and a lower cover layer, which can be formed from the same material or different materials as needed.

The electrode may be made from any conductive material. For example, it may be formed using a metal, a carbon-based material, or an alloy. The electrode may include at least one component selected from the group consisting of magnesium (Mg), molybdenum (Mo), zinc (Zn), tungsten (W), silicon (Si), and their mixtures or alloys. Additionally, the electrode may be formed using a conductive gel material.

If the electrode is made from a conductive material, the present invention does not impose specific limitations on its composition. It is preferable that the electrode material be biodegradable so that it can degrade along with the cover layer or the material of the central support portion. Furthermore, the electrode material may be manufactured in the form of nanowires.

The cover layer may be formed of a polymer capable of sealing the electrode. It is preferable that the polymer possesses characteristics that allow it to fold and unfold effectively while also being biodegradable. The cover layer may be formed using a highly flexible and highly elastic biodegradable polymer. In one embodiment of the present invention, the electronic umbrella device was manufactured by blending PLCL and PLGA polymers. However, the cover layer material of the present invention is not limited to these specific polymers. Any highly flexible, highly elastic biodegradable polymer capable of forming chemical bonds with various functional groups may be suitable. The polymer material may primarily consist of ester-bonded polymers capable of hydrolytic biodegradation and may additionally include urethane-based polymers capable of enzymatic degradation. At least a portion of the cover layer may have an open structure, allowing part of the electrode to be exposed externally. The exposure of the electrode enables it to make close contact with the upper or lower target surface, such as the brain surface or the inner skull wall.

In one embodiment, the branched portion may include a shape-memory polymer that restores its original shape when certain environmental conditions are met.

Before the required environmental conditions are satisfied, the polymer material can maintain an arbitrary temporary shape while behaving flexibly, allowing it to crumple or fold, thereby preventing damage to the device.

The polymer material must be capable of unfolding back to its original shape after being crumpled or folded inside the body. This unfolding process can be designed to function similarly to the self-assembly of copolymers, where the material autonomously returns to its pre-designed shape once exposed to the appropriate external conditions, reverting to its original structure before folding or crumpling. It is particularly preferable for the polymer material to exhibit the properties of a shape-memory polymer.

In one embodiment, the environmental conditions required for activation may include one or more conditions selected from the group consisting of temperature, humidity, pH concentration, light intensity, ultrasound, and electromagnetic fields in the surrounding environment.

These environmental conditions may be physical, chemical, or biological conditions. In this case, the environmental condition may correspond to the presence or absence of a specific component in the external environment. Additionally, it may be the presence or absence of light, ultrasound stimulation, or an electromagnetic field. Furthermore, it may also refer to inherent conditions of the external environment, such as temperature, humidity, or pH concentration.

Such polymer materials are adopted as the primary material forming the matrix of the branched portion in the present invention. The branched portion is a critical component of the electronic umbrella device, as it must restore to its original shape after being injected into a confined space and then expanding into a broader area.

In one embodiment, the branched portion may include a highly flexible polymer with a Young's modulus of at least 20 kPa.

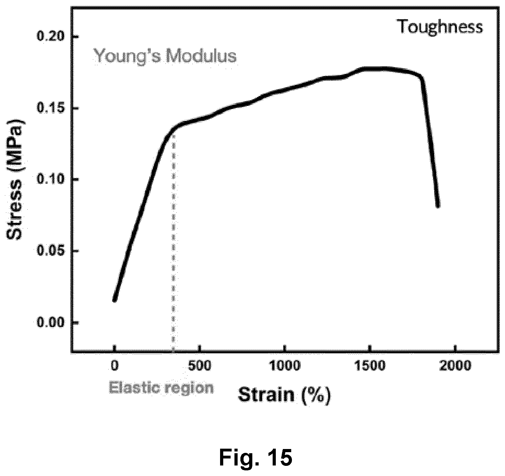

FIG. 15 of the present invention illustrates the results of a Young's modulus experiment conducted to test the polymer material conditions necessary for the electronic umbrella device to unfold effectively in a constrained environment. The inventors confirmed that, to achieve the objective of enabling the crumpled electronic umbrella device to unfold smoothly under limited height conditions without affecting the target surface, a highly flexible polymer material may be required.

In this case, the highly flexible polymer material should not only undergo the intended shape transformation without damage but also gently settle onto the target surface. Preferably, it should have a Young's modulus of at least 20 kPa. More preferably, the polymer material may have a Young's modulus of at least 25 kPa.

If the polymer material is configured with a Young's modulus below 20 kPa, there is a risk that it may not settle smoothly during the unfolding process after implantation or may fail to expand into the intended structure, instead remaining in a folded state.

The branched portion may also include a polymer with a broad elastic range of at least 250% based on Young's modulus. This polymer may not only exhibit ductility but also incorporate polymer components with high elasticity or be configured to include a polymer that simultaneously satisfies both properties.

The polymer material may be a shape-memory polymer that autonomously restores to its original shape when specific conditions are met. However, if functional groups that facilitate shape restoration are added to this polymer, the shape recovery process can be further enhanced. The inventors have focused on the temperature responsiveness of DNA as a functional group for aiding shape restoration.

In one embodiment, the branched portion may further include DNA combined with the polymer, where the DNA may include a hairpin structure or double-stranded DNA.

Additionally, the branched portion may be formed using a DNA-containing material only in specific regions where shape transformation mechanisms need to occur. These specific regions may correspond to areas experiencing high strain during deformation. The designated regions may form distinct sections of the branched portion, separate from other areas.

The DNA has an internal hydrogen bond structure, and in response to the surrounding temperature, reactions such as the unfolding of the hairpin structure or the separation of the double strand may occur. By including DNA, the temperature-responsive structural deformation can be equipped with more precise shape restoration control ability.

The aforementioned DNA is cited as an example where the polymer responds to temperature conditions, causing branch modifications. However, if the shape restoration of the electronic umbrella device occurs under different conditions, other components that assist in the polymer's restoration under those conditions may additionally be included in the electronic umbrella device.

According to one embodiment, the branch portion may form a network structure. In one embodiment, the network structure may include a plurality of chassis portions; arranged radially around the central support portion placed at the center, and support portions; formed to interconnect the chassis portions.

The chassis portion may serve as the main framework of the branch portion, and various device portions may be formed on the chassis portion. The support portion may function to support the chassis portion so that it can be restored to its original shape while the chassis portion folds and unfolds. Additionally, the support portion may also serve as a passage through which multipurpose circuits or electrodes pass. The support portion may be formed in a wavy or cylindrical design and i may also form a curved pattern with a symmetrical structure. The support portion may directly or indirectly participate in the behavior of the chassis portion, such as dispersing strain or facilitating effective unfolding when the electronic umbrella device folds and unfolds.

According to one embodiment, at least a part of the portion where the chassis portion and the chassis support portion are connected may have a device portion formed. The device portion may be formed at the portion where the chassis portion and the support portion are connected, forming a structure electrically connected to the device portion formed on the adjacent chassis portion through the support portion. Also, according to one embodiment, at least a part of the end or intermediate point of the chassis portion may have each device portion formed. That is, the device portion may be formed in any part of the chassis portion of the branch portion, whether it is the end, the portion connected to the support portion, or the portion not connected to the support portion, as determined by the design for its intended purpose.

At this time, according to one embodiment, it may include one or more of a sensor unit, an electrode unit, an electrical stimulation unit, a drug injection unit, an ultrasound transmission unit, a battery unit, and a light transmission unit.

The sensor unit may be interpreted as a general concept and encompass sensors applied to various applications. The electrical stimulation unit may perform a role of promoting the transmission of the target object.

The ultrasound transmission unit may enable the implementation of functions such as stimulation or signal transmission through ultrasound, in addition to the electrical stimulation generated through the electrical stimulation unit, and may thereby enable therapeutic or diagnostic functions.

The light transmission unit may be configured to include a light-transmitting medium, such as a light source, a photothermal device, or an optical fiber. When the optical fiber is included in the device unit, it may be utilized as a cooler to cool specific points of the brain.

The device unit may also be configured to include a battery unit that generates electrical energy autonomously in one embodiment. The battery unit may be formed to include a biodegradable battery.

According to one embodiment, the device unit may be configured such that a single device unit performs multiple functions. It is not necessary for one device unit to perform only one function, and if circuits or electrodes are formed sufficiently small, one device unit may include multiple sensors or multiple electrodes. In addition, it is possible to further include one or more of a drug injection unit, an ultrasound transmission unit, a battery unit, or a light transmission unit simultaneously.

According to one embodiment, at least two or more of the device units may perform different functions. In a specific embodiment, depending on the location in the brain, different stimulations or drug injections may be required. In this case, to perform diagnosis, treatment, and prescription suitable for each location, the device units at different locations may be designed to perform different functions.

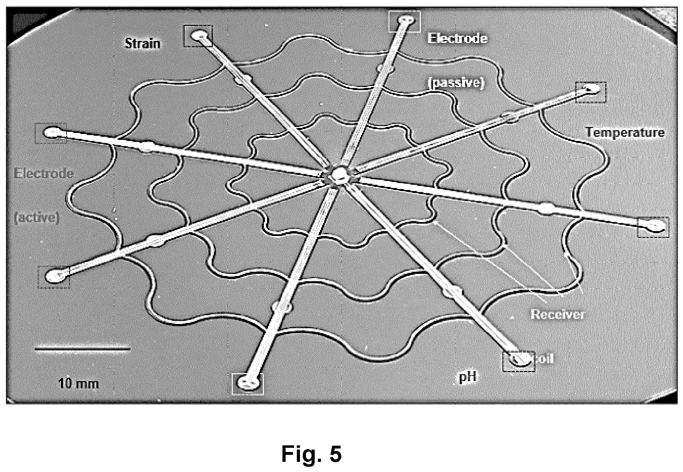

FIG. 5 is an image illustrating an example in which different sensors and electrodes are formed at the device unit formed at the end of the branch portion of the electronic umbrella device according to one embodiment of the present invention. As shown in FIG. 5, it can be confirmed that a strain sensor, a temperature sensor, a pH sensor, a passive electrode, and an active electrode are formed at the respective ends of the chassis portion.

In addition, in the example shown in FIG. 5, it can be confirmed that the support portion connecting each chassis portion is formed in a symmetrical shape having a wavy curve. Although this structure is merely an example, the introduction of such a support portion may enable stable shape transformation even under strain applied to each part during the folding and unfolding process.

The example shown in FIG. 5 is not limited to this case, and each single device unit may include a plurality of various types of sensors, and sensors and electrodes may be formed together. Additionally, a circuit capable of applying drug delivery or electrical stimulation to the brain surface may also be formed together.

According to one embodiment, each chassis portion having device units performing different functions may be assembled to be connected to the central support portion. In a specific embodiment, the respective branches are not manufactured simultaneously with the central support portion, but rather, each branch equipped with the intended device unit at different locations may be individually assembled to the central support portion, forming an integrated structure.

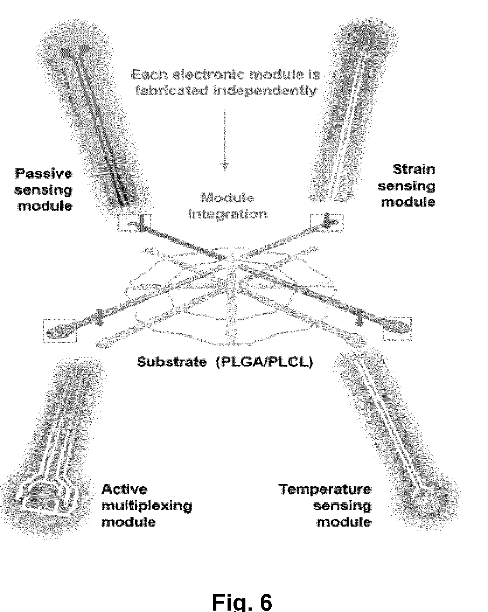

FIG. 6 is a schematic diagram illustrating that different electronic modules may be formed at the device unit formed at the end of the branch portion of the electronic umbrella device according to one embodiment of the present invention. As shown in FIG. 6, each chassis portion may be manufactured in a form that mounts a pre-designed electronic module and is assembled to face the intended direction of the central support portion according to the need and purpose of the electronic device. At this time, the lower cover layer (base substrate) of the electronic module may not only be sealed but also be formed of a material that has flexibility during the folding and unfolding process. In this embodiment, the substrate was manufactured using a polymer material blended with PLGA and PLCL, and in addition to this, various polymer materials with flexibility and elasticity can be used to manufacture the lower cover layer and upper cover layer.

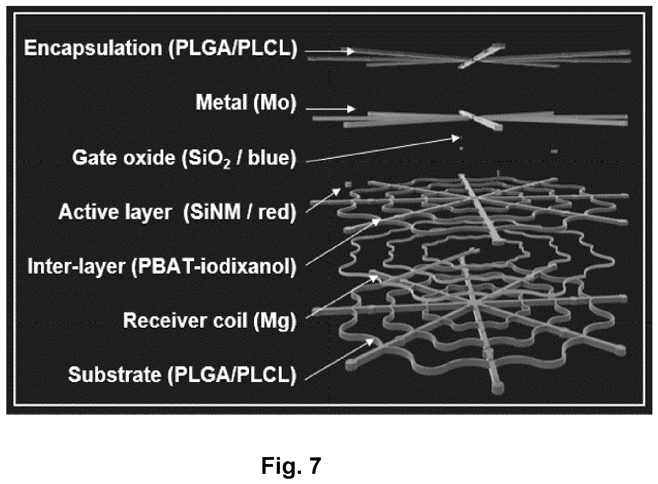

FIG. 7 is a schematic diagram illustrating the laminated structure of the electronic umbrella device according to one embodiment of the present invention.

According to one example, a receiver coil for electronic communication means may be positioned on a lower cover layer (base substrate) made of a flexible material, and a separation layer (inter-layer) that divides the upper layer and lower layer may be formed thereon. An active layer may be formed on the upper part of the separation layer, and above that, a gate oxide layer and an electrode layer may be formed. The topmost layer may include an upper cover layer that is symmetrically formed with the lower base substrate, which can function to seal each of the layers positioned between the lower cover layer and the upper cover layer.

The layers described above are merely examples forming the electronic umbrella device of the present invention, and it is possible to add specific layers as needed, as well as to omit some layers when manufacturing the electronic umbrella device.

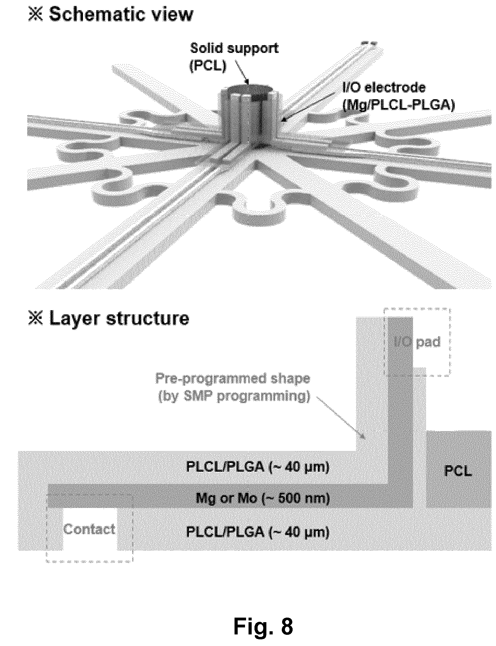

FIG. 8 is a schematic diagram illustrating the central support portion of the electronic umbrella device according to one embodiment of the present invention, showing the solid support that supports the upright structure of the central support portion (upper diagram), and a cross-sectional view illustrating the connection structure between the branch portion and the central support portion of the electronic umbrella device (lower diagram).

In the upper diagram of FIG. 8, the solid support not only serves to physically support the central support portion but also prevents the electronic umbrella device from collapsing or losing its central alignment when the electronic umbrella device is folded and inserted into the injection device or ejected and unfolded from the injection device.

In the lower diagram of FIG. 8, the branch portion supports the central support portion and is depicted as having an "L"-shaped structure that folds upward in the vertical direction (z-axis direction). Such a folded structure can facilitate the formation of a wired connection with other external electronic devices. Of course, the electronic umbrella device of the present invention may also be designed to communicate with other external electronic devices wirelessly. The branch portion may include an electrode line made of Mo or Mg metal material inside and may be encapsulated with a lower cover layer and an upper cover layer. At this time, a part of the lower cover layer may be formed as an open structure, exposing part of the electrode to the outside, which can allow electrical contact with the brain surface or a specific location inside the skull as intended.

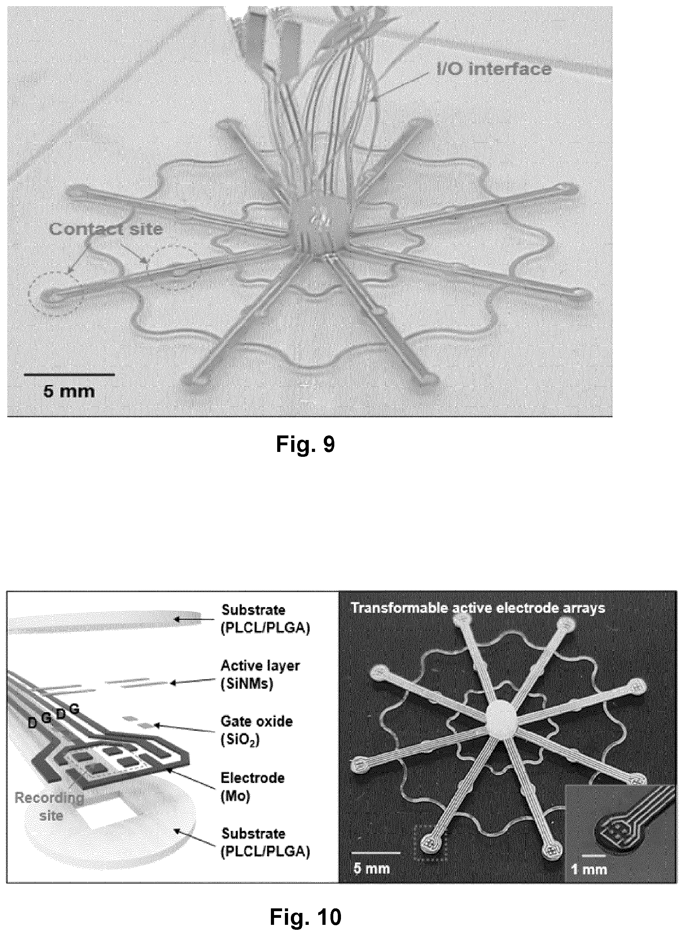

FIG. 9 is an image illustrating the structure in which the electrode is wired and connected to an external electronic device through the folded structure of the central support portion shown in FIG. 8, according to one embodiment of the present invention. In FIG. 9, it can be confirmed that a plurality of device units are formed at the end and intermediate points of the chassis portion. According to some embodiments, multiple device units may be formed at respective points even within a single chassis portion.

FIG. 10 is an image illustrating the actual manufactured form and scale of the electronic umbrella device according to one embodiment of the present invention, showing the laminated structure of the device unit formed at the end of the branch portion and its unfolding into the original shape in response to temperature. In the right image of FIG. 10, it can be observed that each device unit may be formed with a diameter of approximately 1 mm.

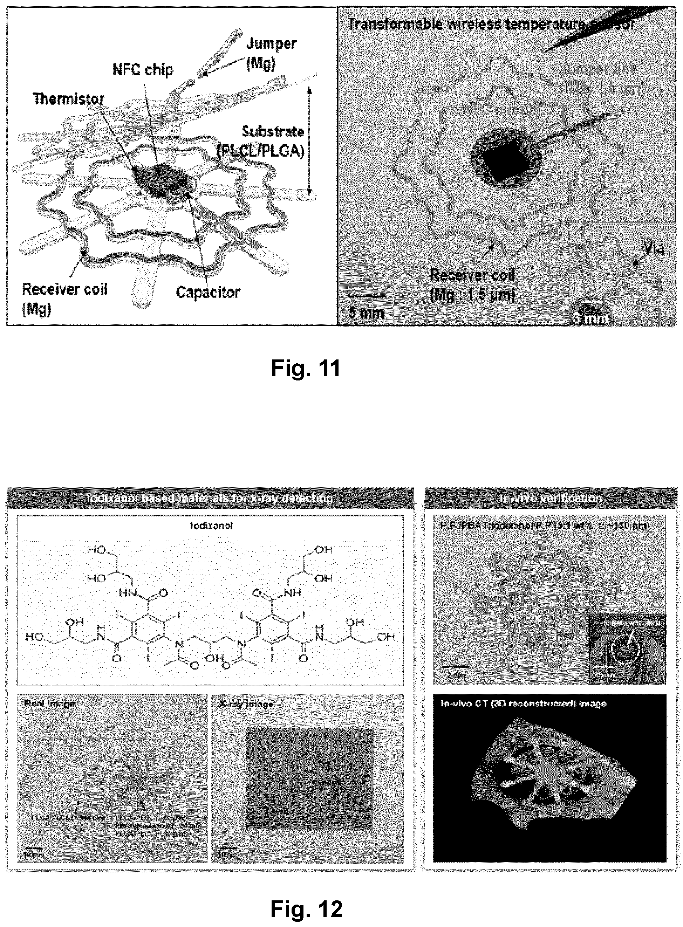

FIG. 11 is a schematic diagram illustrating the laminated structure of the electronic umbrella device with a wireless communication function according to one embodiment of the present invention.

If it is intended to enable the electronic umbrella device to wirelessly transmit and receive signals with external devices, an NFC circuit or capacitor may be mounted on the central support portion. The results presented in FIG. 11 confirm that the electronic umbrella device of the present invention can be designed to wirelessly transmit and receive signals with external electronic devices.

In many experimental examples conducted by the inventors, the folded or crumpled electronic umbrella device, when ejected from the injection device, did not smoothly unfold into the intended shape, resulting in issues such as the central axis collapsing or some branch portions not unfolding and remaining folded.

The inventors conducted various experiments to optimize the shape restoration yield. In one of the experiments, attention was given to the contact angle formed when the end of each chassis portion initially made contact with the attached bottom surface, and the sliding motion according to the contact angle was analyzed. The inventors confirmed that, in order for the shape restoration to occur effectively under the limited height conditions intended in the present invention, the landing angle of the chassis portion's bottom surface, rather than a support portion that can be easily unfolded due to its curved form, is important. Through this experiment, it was concluded that the initial contact angle between each chassis portion and the bottom surface (brain surface) must be formed at a certain level.

The chassis contact angle mentioned in the present invention refers to the angle formed between the surface to which the device is intended to adhere and the surface when the device comes into contact, and it is a critical factor directly related to successful shape restoration.

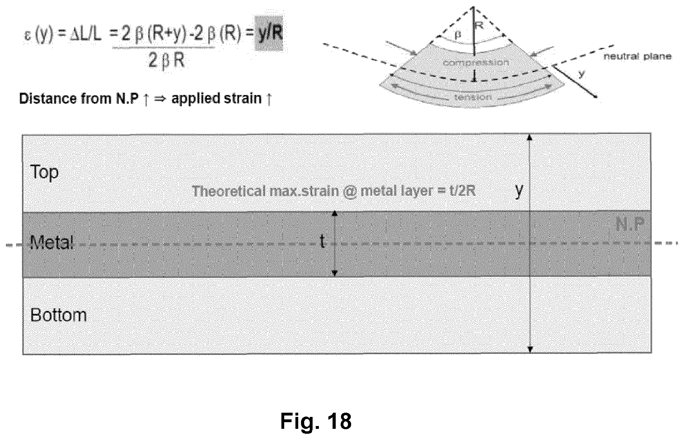

FIG. 18 shows the simulation results analyzing the factors that influence the effective unfolding from a crumpled or folded 3D structure to a 2D structure in the electronic umbrella device according to one embodiment of the present invention. As confirmed in the experimental example shown in FIG. 18, the inventors conducted various experiments on the ejection and unfolding of electronic umbrella devices to investigate the direction in which the electronic umbrella device slides, and what structure and size of the support portion's radius enable the desired contact angle to be achieved.

As shown in the image of FIG. 18, when the contact angle is formed to be 70 degrees or less, a forward sliding motion, as seen at the upper right, occurs, and sufficient space for shape restoration is secured outward, resulting in successful shape restoration. On the other hand, when an angle exceeding 70 degrees is formed, a backward sliding motion or other unnecessary mixed sliding motions occur, causing each chassis to roll inward or crumple. When the chassis folds inward in this way, they have a very limited restoration space, leading to buckling and entanglement, which may result in unsuccessful shape restoration.

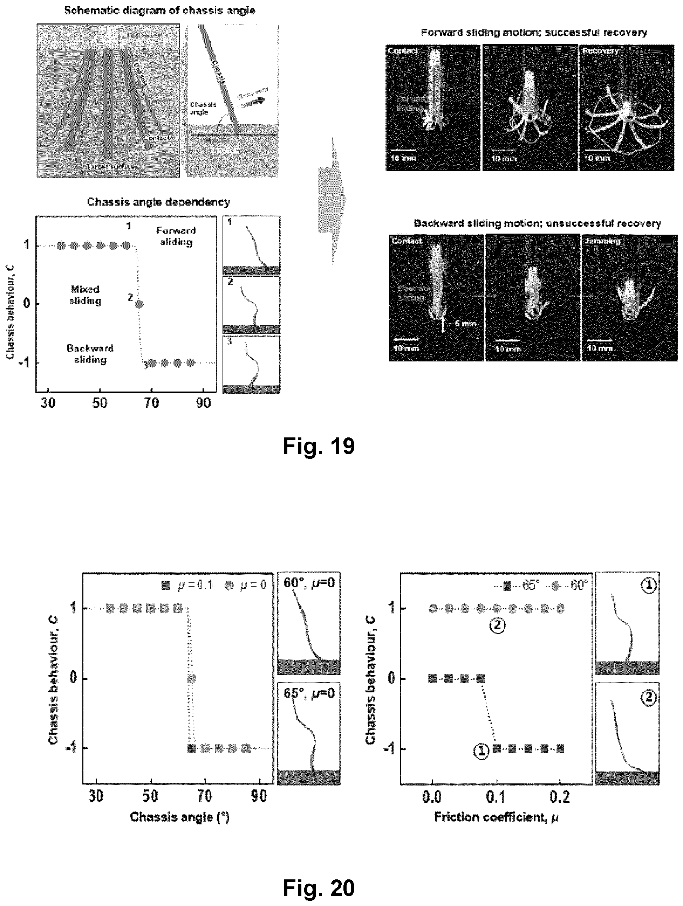

FIG. 19 is an image illustrating the problem of compensation (reduction) of the chassis angle at the end of the chassis portion and the resulting mechanism change when considering the friction of the brain surface in the electronic umbrella device according to one embodiment of the present invention.

Additionally, when the friction factor on the brain surface is considered, the desirable range of the contact angle for the chassis end to appropriately slide forward may be somewhat reduced.

FIG. 20 is an image illustrating the problem of compensation (reduction) of the chassis angle at the end of the chassis portion and the resulting mechanism change when considering the friction of the brain surface in the electronic umbrella device according to one embodiment of the present invention.

According to one embodiment, when the bio-implantable electronic device is injected in a state where the branch portion first contacts the brain surface, the contact angle at which the end of the chassis portion initially contacts the target object is preferably 70 degrees or less, and more preferably 65 degrees or less. The contact angle may be 0.1 degrees or more. If the contact angle is less than 0.1 degrees, entry itself becomes difficult when considering minimally invasive procedures. If the contact angle exceeds 70 degrees, it may cause damage to the brain surface or fail to achieve effective outward sliding of the electronic device, leading to crumpling or folding issues, which may result in the shape not being restored as intended or even damage to the device.

At this time, the contact angle is determined as the acute angle at which the end of the chassis portion enters the target object (e.g., brain surface), as shown in FIGS. 19 and 20.

Additionally, when considering the friction of the brain surface, compensation of the chassis angle may occur, reducing the appropriate range for forward sliding motion. Preferably, the contact angle may be 60 degrees or less.

Meanwhile, according to one embodiment, the bio-implantable electronic device may include an X-ray marker.

FIG. 12 shows an image of the electronic umbrella device according to one embodiment of the present invention, manufactured to include an X-ray marker, along with an X-ray image of the manufactured example. When manufacturing the electronic umbrella device of the present invention, if at least a part of each layer includes an X-ray marker material, it is possible to easily check whether the electronic umbrella device has been properly implanted, unfolded, or attached to the desired position, as shown in FIG. 12. In this embodiment, Iodixanol-based compounds were used as the X-ray marker and X-ray imaging was performed, but in the concept of the present invention, various X-ray marker materials can be used. Furthermore, it is also possible to include various position detection marker materials that serve as identifiers for various diagnostic devices.

According to one embodiment, the central support portion forms a column-shaped shaft structure in the z-axis direction, and the central support portion may include means for enabling wireless communication with the outside or a conductive line for enabling wired communication. The means for enabling wireless communication is not particularly limited in the present invention, and an NFC chip may be used as an example. When the electronic umbrella device is configured to be connected by wire, the conductive line may pass vertically (in the z-axis direction) through the inside of the central support portion or an adjacent area and extend to the outside of the skull.

According to one embodiment, the central support portion may include a solid support that supports the upright structure of the central support portion. The solid support may be formed in an "L" shape as shown in FIG. 8, forming a bent structure from the x-y plane to the z-axis direction, connecting and supporting between the branch portion and the central support portion.

The electronic umbrella device proposed for minimally invasive procedures according to another embodiment of the present invention is a bio-implantable electronic device that includes a central support portion; and a branch portion including a plurality of chassis portions extending from the central support portion. After being crumpled into a 3D structure in the z-axis direction outside the body with a height of 5 mm or less and injected into the internal environment, it unfolds into a 2D structure in the x-y axis direction within the body, thereby enabling minimally invasive procedures.

According to one embodiment, the electronic umbrella device may have an unfolded area that is more than 10 times larger than the folded area. The electronic umbrella device of the present invention may have an area on the x-y plane in the fully unfolded state that is more than 10 times larger than the cross-sectional area of the central support portion. The area in the folded state may be equal to or smaller than the cross-sectional area of the central support portion. The folded area may be based on the cross-sectional area of the injection part of the injection device (for example, the cross-sectional area of the syringe outlet). According to embodiments of the present invention, the area ratio can be extended to 100 times or more without difficulty. In one example, the unfolded area can be expanded to a diameter of approximately 15 mm.

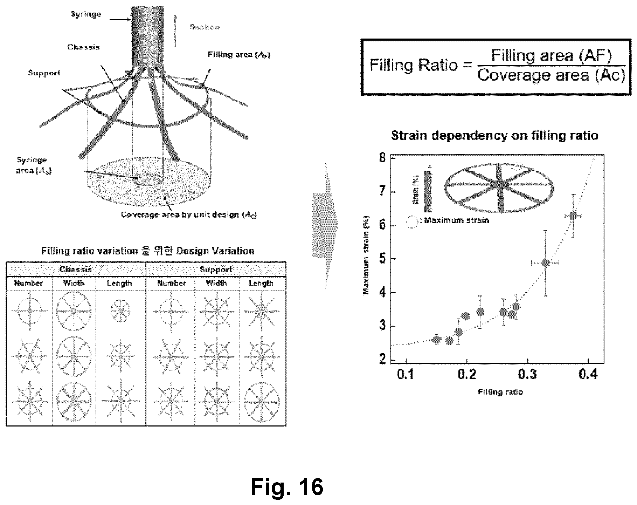

FIG. 16 is a simulation image showing the results of exploring the optimal structure of the chassis portion and support portion to enable effective unfolding of the electronic umbrella device when discharged from the injection device under restricted conditions, according to one embodiment of the present invention.

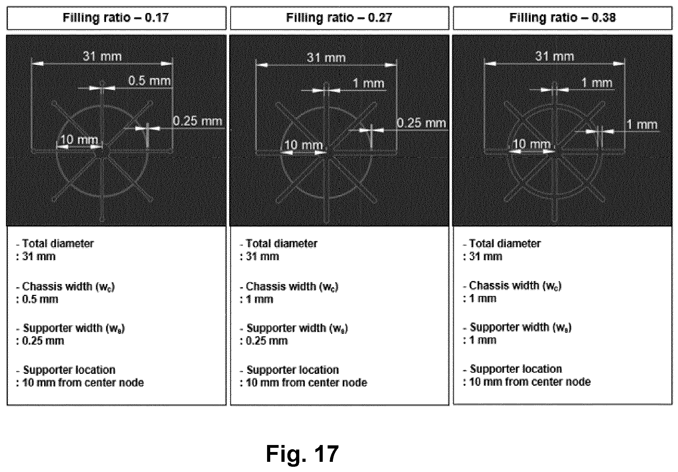

FIG. 17 is an image showing the filling ratio values according to the design of each part of the electronic umbrella device according to one embodiment of the present invention.

The inventors designed various structures of the chassis portion and chassis support portion (branch portion structure) to quantify the strain applied to the electronic umbrella device discharged from the injection device and analyzed the strain at each position. By varying the number, width, and length of the chassis portion and chassis support portion, the maximum strain values occurring under each condition were compared based on the coverage area (the maximum area when the device is fully unfolded) and the filling area (the actual device area, which is the sum of the cross-sectional areas of the central support portion and branch portion).

Through this, the inventors newly derived a parameter called the Fraction of Filling area, which can determine the optimal value for the electronic umbrella device discharged from the injection device to effectively unfold under the height condition of 5 mm or less.

*Filling Ratio = Fraction of Filling area = Filling Area (AF) / Coverage Area (Ac)

The inventors defined the optimal design structure that allows 2D - 3D - 2D transformation under restricted conditions as the Filling Ratio (Fraction of Filling area).

In the present invention, the Filling Ratio is defined as the ratio of the actual device area (filling area) to the total cover area (coverage area) occupied by the device. The Filling Ratio can be adjusted by varying the number, width, and length of the chassis portions and support portions. Through FEA simulations conducted by varying the Filling Ratio, it was confirmed that as the Filling Ratio increases, the maximum strain applied to the device also increases.

The Filling Ratio may represent the optimal design structure that prevents electronic components formed on the polymer material of the electronic umbrella device from becoming brittle or breaking during the folding and unfolding process. The Filling Ratio may be defined as a numerical value close to the limit at which the behavior intended in the present invention is achievable without causing damage to the electronic components, based on the material or structure of the electronic components.

According to one embodiment, the electronic umbrella device may have a Filling Ratio of 0.6 or less.

Also, the Filling Ratio may be 0.05 or more. If it is less than 0.05, the area of the electronic umbrella device for mounting electronic components such as electrodes becomes too small, making it difficult to effectively utilize the electronic umbrella device.

On the other hand, if the Filling Ratio exceeds 0.6, the strain applied to the device becomes excessively large, potentially causing cracks or damage to the polymer material or electrode material that forms the base of the device.

Preferably, the Filling Ratio may be 0.4 or less, and more preferably, it may be formed within the range of 0.1 to 0.38.

FIG. 18 is a schematic diagram illustrating the cross-sectional structure of the lower cover layer and upper cover layer around the electrode of the branch portion of the electronic umbrella device according to one embodiment of the present invention. In the embodiment shown in FIG. 18, it was confirmed that when the lower cover layer and upper cover layer are formed with the same cross-sectional thickness, a neutral plane is formed, thereby minimizing the strain applied to the electrode and electronic component layers.

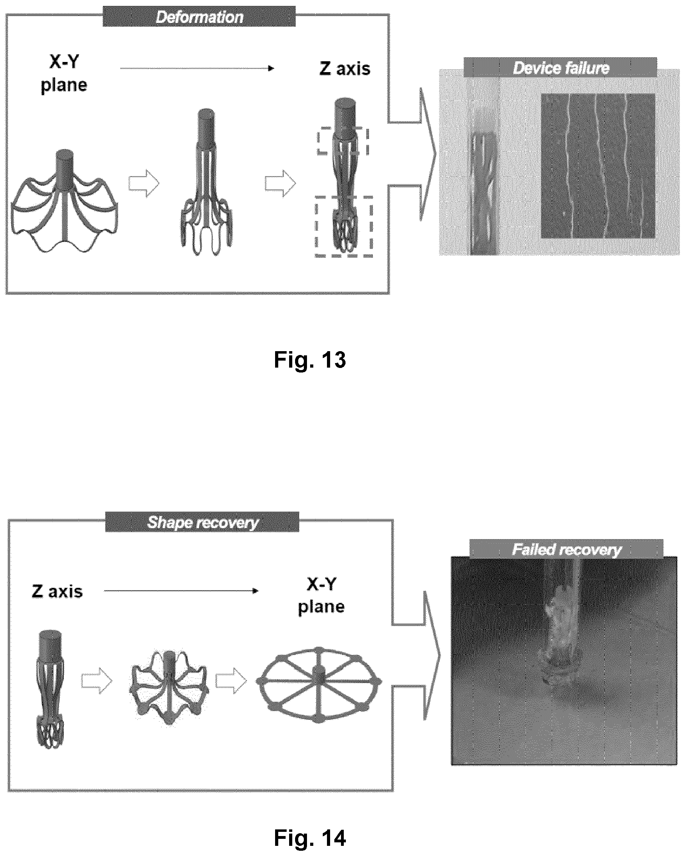

FIG. 13 is an FEA simulation image and an actual imaging image showing that the electronic umbrella device according to one embodiment of the present invention may experience excessive strain when first injected into the injection device, which may lead to concerns about device damage and the formation of cracks. At this time, the electronic umbrella device transforms from a two-dimensional structure on the x-y plane into a three-dimensional columnar structure, folding or crumpling to a size approximately equivalent to the diameter of the central support portion.

The electronic umbrella device proposed for minimally invasive procedures according to another embodiment of the present invention may have a structure in which, when the branch portion is injected in the direction of first contacting the brain surface, the highest chassis support portion closest to the central support portion among the plurality of chassis support portions experiences the greatest strain. The electronic umbrella device proposed in the present invention may have a structure in which, when the branch portion first enters the target surface, the greatest strain is applied to the highest chassis support portion among the chassis support portions formed to connect the space between the chassis portions rather than the chassis portions themselves. At this time, if only one chassis support portion is formed to connect the ends of the chassis portions, the maximum strain may be applied to the terminal chassis support portion connecting the ends of the chassis portions (refer to FIG. 13).

FIG. 14 is an FEA simulation image and an actual imaging image showing the electronic umbrella device according to one embodiment of the present invention, where the device is discharged from the injection device to the outside but fails to unfold as planned. In this case, the electronic umbrella device, after being discharged from the injection device, must smoothly unfold from the folded or crumpled three-dimensional column structure back to the original two-dimensional structure on the x-y plane. However, even when using shape-memory materials and satisfying temperature conditions, it was confirmed through this experiment that precise design of various conditions is necessary for the electronic umbrella device to be restored to its original structure under limited height conditions.

From the simulations shown in FIGS. 13 and 14, it was analyzed that significant strain is applied to the support portion connecting the central support portion and the branch portion, as well as to the chassis support (support) portion connecting between the chassis portions.

The electronic umbrella device proposed for minimally invasive procedures according to another embodiment of the present invention is a bio-implantable electronic device comprising a central support portion; and a branch portion including a plurality of chassis portions extending from the central support portion. When entering the environment between the cranial inner wall and the brain surface, the branch portion unfolds and comes into contact with the brain surface to function. The chassis portion consists of multiple layers stacked and then encapsulated, and may include at least three layers: a lower cover layer, an electrode, and an upper cover layer.

In this case, as described above, the lower cover layer and the upper cover layer perform the role of encapsulating the electrode and are formed of a material that possesses both superflexibility and high elasticity. The selection of the materials for the lower and upper cover layers is an important factor in ensuring that the electronic umbrella device of the present invention can crumple, fold, and then unfold without damage.

As previously described and shown in FIG. 18, forming the lower and upper cover layers symmetrically can minimize the strain on the sealed electrode or electronic component positioned in the center. Therefore, it is desirable to form the lower and upper cover layers with the same thickness.

According to one embodiment, the branch portion may further include one or more of the following layers: an active layer, an inter-layer, a receiver coil layer, and a gate oxide layer. The electronic umbrella device of the present invention can be configured by incorporating various layers as needed. Examples of such configurations are illustrated in FIGS. 7, 10, and 11.

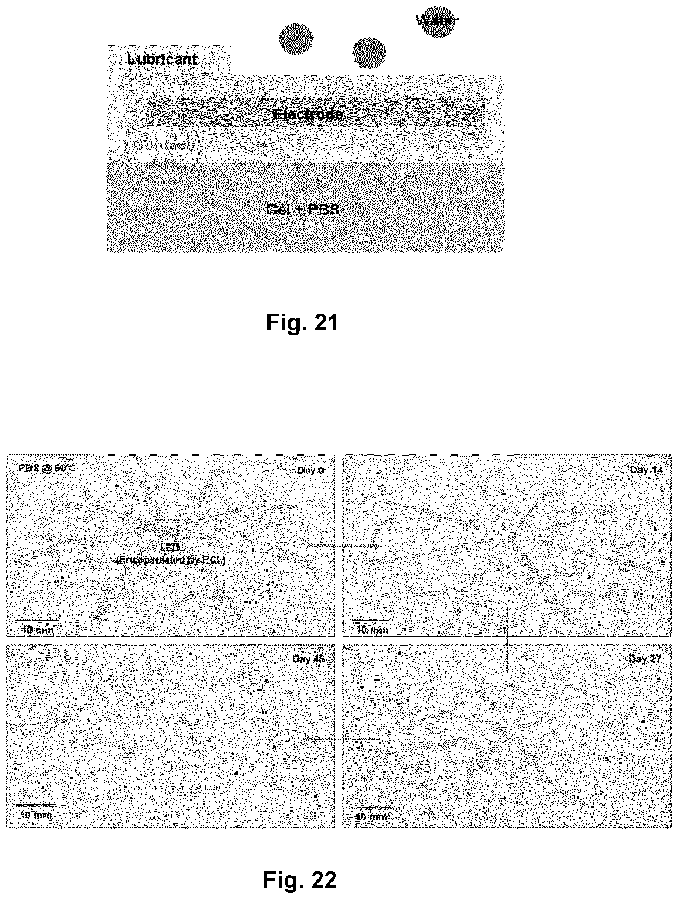

FIG. 21 is a schematic diagram illustrating that the electronic umbrella device according to one embodiment of the present invention can unfold more smoothly when a lubricant is present. If an appropriate lubricant is injected together with the injection device or introduced into the body using a separate tool, the electronic umbrella device of the present invention will be able to unfold more easily. However, there is also a risk that the presence of the lubricant may cause the device to deviate from the intended position, preventing the component from adhering to the correct point. Therefore, it has been recognized that research is needed to determine the optimal lubricant conditions.

Details related to this will be further discussed when describing the injection method of the electronic umbrella device.

FIG. 22 shows an image illustrating the result of the electronic umbrella device according to one embodiment of the present invention undergoing biodegradation after an appropriate period of time as pre-designed. In this embodiment, the electronic umbrella device gradually decomposes after 2 weeks, and after 45 days, most of the device has decomposed into strands within 10 mm in length. The degradation rate of such an electronic umbrella device can be adjusted as needed by designing the material composition with a combination of various biodegradable materials.

## MANUFACTURING METHOD OF THE ELECTRONIC UMBRELLA DEVICE

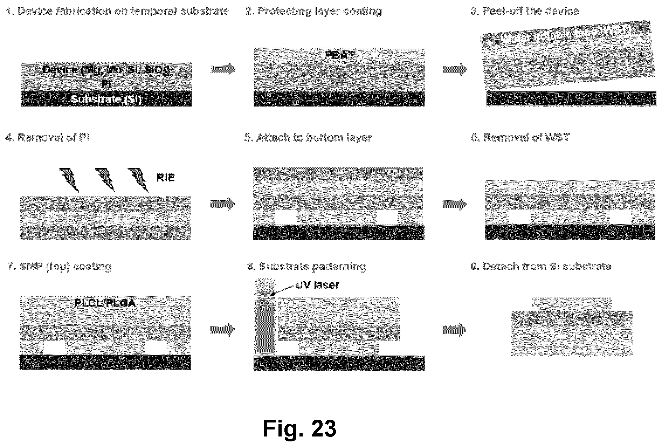

Hereinafter, the manufacturing method of the electronic umbrella device according to the present invention will be described with reference to FIGS. 23 and 24.

The electronic umbrella device of the present invention can be manufactured using all methods generally applicable in the technical field, and the present invention does not particularly limit the manufacturing method. However, the content described below aims to explain that the electronic umbrella device according to a specific embodiment can be manufactured through somewhat unique methods proposed herein.

In another embodiment of the present invention, the manufacturing method of the electronic umbrella device for minimally invasive surgery does not involve a one-step process such as molding. Instead, the electronic umbrella device of the present invention can be manufactured through a multi-step lamination process similar to semiconductor processes. The characteristic aspect of the manufacturing method of the electronic umbrella device according to the present invention is clearly revealed in the manufacturing process of the branch portion. FIGS. 21 and 22 sequentially illustrate and explain the manufacturing process of the chassis portion of the branch.

FIG. 23 shows a process diagram illustrating the sectional structure of each step in the manufacturing process of the chassis portion of the electronic umbrella device according to one embodiment of the present invention. In this embodiment, unlike stents or other surgical tools that utilize shape memory alloys to restore the intended shape within the body, the electronic umbrella device can be a sealed electronic device that encapsulates electrodes and circuits within a polymer. Therefore, unlike conventional surgical tools using alloys or metals, the device can be manufactured using lamination processes, photolithography processes, laser processes, and the like, commonly used in semiconductor processing. However, the method presented in this figure is merely an example of manufacturing the electronic umbrella device of the present invention, and the technical concept of the present invention is not limited thereto.

Also, the chassis portion can be attached to the central support after forming the device part according to the intended purpose.

In this case, the aforementioned manufacturing method of the electronic umbrella device may include a step of assembling a plurality of prepared chassis portions to the central support.

Fig. 24 illustrates a process diagram showing the steps of attaching the chassis part of the electronic umbrella device according to an embodiment of the present invention to the lower substrate and connecting it to the central support. Through the image of Figure 24, it can be confirmed that the chassis part is attached based on the central support and folded in an "L" shape to extend in the vertical direction (z-axis direction).

In the embodiment of the present invention, unlike stents or other surgical tools that restore to a predetermined shape within the body using shape memory alloys, the electronic device of the present invention can be a sealed electronic device that encapsulates electrodes and circuits within a polymer. Therefore, unlike conventional surgical tools that use alloys or metals, it can be manufactured using processes commonly used in semiconductor fabrication, such as stacking processes, photoresist processes, and laser processes. However, the method presented in this drawing is merely an example of manufacturing the electronic umbrella device of the present invention, and the technical idea of the present invention is not limited to this.

Although this method is a simple example of combining the chassis part with the central support, when the chassis part is combined with the central support in this way, the chassis part may include: a step of preparing a lower substrate; a step of forming a lower cover layer on the lower substrate; a step of forming the central support on the lower cover layer; a step of forming a laminate of prepared electrodes and an upper cover layer on the lower cover layer; and a step of folding one side of the lower cover layer, electrode, and upper cover layer upward based on the central support.

The manufacturing method of the implantable electronic device proposed in another embodiment of the present invention includes forming a laminated structure comprising the lower cover layer, electrode, and upper cover layer on the branch part, and the process of forming the laminated structure may include a step of designing the structure of each layer through a laser process. The process of forming the laminated structure through the laser process is also well illustrated in Figs. 21 and 22.

## METHOD FOR IMPLANTATION OF ELECTRONIC UMBRELLA DEVICE IN THE BODY

An embodiment of the present invention proposed as another aspect to achieve the aforementioned technical objective relates to the method for implanting an electronic umbrella device for minimally invasive surgery in the body.

The method for implantation of the implantable electronic device proposed in an embodiment of the present invention involves the implantable electronic device entering the environment between the skull inner wall and the brain surface, whereby the branch part unfolds and comes into contact with the brain surface to function. The implantable electronic device comprises a central support part and a branch part that extends from the central support part and includes multiple chassis parts. The method includes the following steps: folding the implantable electronic device into the injection device; injecting the folded implantable electronic device through a hole drilled in the skull; the implantable electronic device unfolding inside the skull and settling on the brain surface; and the implantable electronic device operating on the brain surface.

According to one embodiment, the step in which the implantable electronic device unfolds inside the skull and settles on the brain surface may include the step of the branch part of the implantable electronic device autonomously unfolding within the intracranial environment.

According to one embodiment, the step of folding the implantable electronic device into a sealed injection device that includes a narrow closed space may further include the step of injecting a lubricant into the sealed injection device. The lubricant not only facilitates smooth folding and insertion of the implantable electronic device into the sealed injection device but also allows the implantable electronic device to smoothly unfold and slide onto the brain surface when deployed, enabling it to be placed on the brain surface without resistance. In this context, by appropriately selecting the material (including coating material) of the part of the electronic umbrella device that comes into contact with the brain surface, it is possible, according to one embodiment, to sufficiently reduce the coefficient of friction.

As previously explained, the lubricant can help prevent the electronic umbrella device from being damaged when entering or exiting the injection device. Additionally, the lubricant can enable the device to glide smoothly on the surface of the target area, thereby reducing the frictional force applied to the target surface.

In the embodiments of the present invention, the lubricant used is a glycerol-based organic compound, but it is not limited to such a material. Any lubricant that is commonly used for surgical purposes and is harmless to the human body can be appropriately selected and used.

According to one embodiment, the step of the bioimplantable electronic device unfolding and positioning on the brain surface may include ensuring that the bioimplantable electronic device adheres well to the intended spot on the brain surface by means of an adhesive. It may be beneficial to have an additional adhesive besides the aforementioned lubricant. The adhesive can serve to ensure that the electronic umbrella device adheres well to the target surface as intended.

In addition to the aforementioned lubricant and adhesive, it may also be beneficial to coat the electronic umbrella device with a waterproof material to ensure proper functioning even in a moisture-rich environment. Especially for the open structure of the aforementioned lower cover layer, applying a waterproof coating that prevents moisture from entering before adhering to the target surface may be considered. Moreover, if the coating is made of a material that disappears after a certain period, the electronic umbrella device can operate more effectively.

**IN-VIVO EXPERIMENT**

The inventors conducted in-vivo experiments with various electronic umbrella devices manufactured using the methods described above to test whether the intended purpose of the invention was properly implemented in the in-vivo environment.

Figs. 25 to 27 show the in-vivo experimental results of the electronic umbrella device according to an embodiment of the present invention, including images and graphs.

Fig. 25 shows experimental images and graphs measuring signals transmitted from each device part when the respective device parts of the electronic umbrella device, according to one embodiment of the present invention, are attached to different positions on the brain surface of an animal.

Fig. 26 shows a graph displaying the temperature signals received after manufacturing the electronic umbrella device, according to one embodiment of the present invention, to enable wireless communication through NFC, implanting it on the brain surface of an animal, and then controlling the temperature by turning an IR lamp on and off. Fig. 24 demonstrates that there is no significant difference between the control group experiment using a commercial temperature sensor and the experiment using the electronic umbrella device according to the present invention.

Fig. 27 shows an image capturing the gradual biodegradation process of the electronic umbrella device, according to one embodiment of the present invention, after being implanted on the surface of an animal for 35 days. The image in Fig. 27 clearly shows that the biodegradation progresses as intended within the targeted time.

The above description merely provides examples for explaining the technical ideas of the present invention. Those skilled in the art to which the present invention pertains will understand that various changes and modifications are possible without departing from the essential characteristics of the present invention. Therefore, the embodiments disclosed herein are for illustrative purposes and are not intended to limit the technical scope of the present invention. The protection scope of the present invention should be interpreted according to the claims, and all technical concepts within the equivalent scope should be construed as being included within the rights of the present invention.

**Claims**

1.    A bio-implantable electronic device comprising:

   a central support portion; and
   a branch portion extending from the central support portion;
   wherein, when entering the environment between the inner wall of the skull and the brain surface, the branch portion unfolds, contacts the brain surface, operates, and then biodegrades to be discharged outside the body, characterized as an electronic umbrella device for minimally invasive procedures.

2.    The electronic umbrella device for minimally invasive procedures according to Claim 1,

   wherein the branch portion comprises an electrode; and a cover layer covering the electrode from above and below;
   wherein the cover layer has an open structure that includes a partially exposed area of the electrode.

3.    The electronic umbrella device for minimally invasive procedures according to Claim 1,
   wherein the branch portion includes a shape-memory polymer that restores its shape when conditions of the surrounding environment are met.

4.    The electronic umbrella device for minimally invasive procedures according to Claim 3,
   wherein the conditions of the surrounding environment include one or more conditions selected from the group consisting of temperature, humidity, pH concentration, light intensity, ultrasound, and electromagnetic field.

5.    The electronic umbrella device for minimally invasive procedures according to Claim 1,
   wherein the branch portion includes a high-elasticity and super-flexibility polymer having a wide elastic region of 250% or more based on the Young's modulus and a modulus of elasticity of 20 kPa or more.

6.    The electronic umbrella device for minimally invasive procedures according to Claim 5,

   wherein the branch portion further includes DNA conjugated to the polymer,
   and the DNA includes a hairpin structure or double-stranded DNA.

7. The electronic umbrella device for minimally invasive procedures according to Claim 6,
wherein the branch portion is formed of a material that includes DNA conjugated to the polymer only in a partial region.

8. **The** electronic umbrella device for minimally invasive procedures according to Claim 1,
wherein the branch portion forms a network structure.

9. **The** electronic umbrella device for minimally invasive procedures according to Claim 8,
wherein the network structure comprises:

   a plurality of chassis portions radially spreading from the central support portion; and
   chassis support portions formed to connect between the chassis portions.

10. **The** electronic umbrella device for minimally invasive procedures according to Claim 9,

   wherein when the biological implantable electronic device is in a bundled state and the branch portion is inserted in a direction to first contact the brain surface,
   the contact angle between the terminal end of the chassis portion and the target is 70 degrees or less.

11. The electronic umbrella device for minimally invasive procedures according to Claim 9,
wherein at least a part of the connection portion between the chassis portion and the chassis support portion is formed with a device portion.

12. The electronic umbrella device for minimally invasive procedures according to Claim 9,
wherein at least some of the terminal and intermediate points of the chassis portion are formed with respective device portions.

13. The electronic umbrella device for minimally invasive procedures according to Claim 12,
wherein the device portion includes one or more selected from a sensor unit, electrode unit, electrical stimulation unit, drug injection unit, ultrasonic transmission unit, battery unit, and optical transmission unit.

14. The electronic umbrella device for minimally invasive procedures according to Claim 12,
wherein the device portion is configured such that a single device portion performs multiple functions.

15. The electronic umbrella device for minimally invasive procedures according to Claim 12,
wherein at least two of the device portions perform different functions.

16. The electronic umbrella device for minimally invasive procedures according to Claim 15,
wherein each of the chassis portions having device portions performing different functions is assembled to be connected to the central support.

17. The electronic umbrella device for minimally invasive procedures according to Claim 1,
wherein the bioimplantable electronic device includes a position detection marker.

18. The electronic umbrella device for minimally invasive procedures according to Claim 1,
wherein the central support forms a shaft structure in the z-axis direction in the shape of a pillar, and the central support includes means for enabling wireless communication with the outside or a conductive line that allows wired communication to pass through.

19. The electronic umbrella device for minimally invasive procedures according to Claim 18,
wherein the central support includes an auxiliary support that supports the standing structure of the central support.

20. A bioimplantable electronic device,

   comprising a central support; and
   a branch part including a plurality of chassis portions extending from the central support,
   wherein the device, when in vitro, is in a bundled 3D structure in the z-axis direction with a height of 5 mm or less, and after being injected into the in vivo environment, unfolds into a 2D structure in the x-y axis direction, enabling minimally invasive procedures.

21. The electronic umbrella device for minimally invasive procedures according to Claim 20,
wherein the area ratio of the expanded state to the folded state of the electronic umbrella device is 10 or more.

22. The electronic umbrella device for minimally invasive procedures according to Claim 20,
wherein the electronic umbrella device has a filling ratio of 0.6 or less.

23. A bioimplantable electronic device,

comprising a central support; and
a branch part including a plurality of chassis portions extending from the central support;
a plurality of chassis support parts configured to connect the plurality of chassis portions to each other;
wherein, when the device enters the environment between the inner skull wall and the brain surface, the branch part expands and contacts the brain surface to operate,
wherein, when the branch part is injected in the direction of first contacting the brain surface, the structure is such that the uppermost chassis support closest to the central support among the plurality of chassis supports is subjected to the most strain.

24. A bioimplantable electronic device comprising:

a central support; and
a branch portion including a plurality of chassis portions extending from the central support;
wherein, when inserted into the environment between the inner wall of the skull and the brain surface, the branch portion unfolds and contacts the brain surface to operate;
wherein the chassis portion is formed by stacking a plurality of layers and subsequently encapsulating them,
wherein the chassis portion includes at least three layers comprising a lower cover layer, an electrode, and an upper cover layer,
an electronic umbrella device for minimally invasive procedures.

25. The device according to claim 24,
wherein the chassis portion further comprises at least one additional layer selected from the group consisting of:

an active layer,
an inter-layer,
a receiver coil layer, and
a gate oxide layer,
an electronic umbrella device for minimally invasive procedures.

26. A method for manufacturing a bioimplantable electronic device,

wherein the bioimplantable electronic device, when entering the environment between the inner skull wall and the brain surface, the branch part expands and contacts the brain surface to operate,
comprising:

a central support; and
a branch part including a plurality of chassis portions extending from the central support;
the branch part forming a stacked structure including a lower cover layer, electrode, and upper cover layer;
the process of forming the stacked structure comprising a step of designing each layer structure through a laser process,
a method for manufacturing an electronic umbrella device for minimally invasive procedures.

27. A method for manufacturing a bioimplantable electronic device,

wherein the bioimplantable electronic device, when introduced into the environment between the inner skull wall and the brain surface, unfolds and comes into contact with the brain surface to operate,
comprising:

a central support portion; and
a branch part comprising a plurality of chassis portions extending from the central support portion;

wherein the chassis portion comprises:

a step of preparing a lower substrate;
a step of forming a lower cover layer on the lower substrate;
a step of forming the central support portion on the lower cover layer;
a step of forming a laminated structure of a prepared electrode and an upper cover layer on the lower cover layer;
a step of folding up one side of the lower cover layer, the electrode, and the upper cover layer relative to the central support portion;
wherein the method is configured for manufacturing an electronic umbrella device for minimally invasive procedures.

28. A method for manufacturing a bioimplantable electronic device,

wherein the bioimplantable electronic device, when introduced into the environment between the inner skull wall and the brain surface, unfolds and comes into contact with the brain surface to operate, comprising:

a central support portion; and
a branch part comprising a plurality of chassis portions extending from the central support portion;
wherein the branch part is manufactured through a process of forming a laminated structure comprising a lower cover layer, an electrode, and an upper cover layer;
wherein the process of forming the laminated structure includes a step of designing the structure of each layer through a laser processing method;
wherein the method is configured for manufacturing an electronic umbrella device for minimally invasive procedures.

29. A method for intracorporeal injection of a bioimplantable electronic device,

wherein the bioimplantable electronic device, when introduced into the environment between the inner skull wall and the brain surface, unfolds and comes into contact with the brain surface to operate, comprising:

a central support portion; and
a branch part comprising a plurality of chassis portions extending from the central support portion;
a step of folding the bioimplantable electronic device into an injection device;
a step of injecting the bioimplantable electronic device through a hole drilled in the skull;
a step of allowing the bioimplantable electronic device to unfold within the inner skull and seat on the brain surface; and
a step of operating the bioimplantable electronic device on the brain surface;
wherein the method is configured for intracorporeal injection of an electronic umbrella device for minimally invasive procedures.

30. The method according to claim 29,
wherein the step of the bioimplantable electronic device unfolding and seating on the brain surface comprises:

a step of allowing the branch part of the bioimplantable electronic device to spontaneously unfold within the inner skull environment,
a method for intracorporeal injection of an electronic umbrella device for minimally invasive procedures.

31. The method according to claim 29,
wherein the step of folding the bioimplantable electronic device into a closed injection device with a narrow cavity comprises:

a step of injecting a lubricant into the closed injection device together with the bioimplantable electronic device;
wherein the lubricant ensures that the bioimplantable electronic device, when unfolding and seating on the brain surface, smoothly unfolds and slides onto the brain surface,
a method for intracorporeal injection of an electronic umbrella device for minimally invasive procedures.

32. The method according to claim 29,
    wherein the step in which the bioimplantable electronic device unfolds and seats on the brain surface comprises:

    attaching the bioimplantable electronic device securely to the designated spot on the brain surface using an adhesive,
    a method for intracorporeal injection of an electronic umbrella device for minimally invasive procedures.

Fig. 1

→ Elastomeric behaviour results in
*normal force* against the tube wall

→ *Frictional force* arises against the
injection direction which results in device
failure

**Fig. 2**

Needs of overcoming limited space for successful injection

**Fig. 3**

Fig. 4

Fig. 5

Fig. 6

Fig. 7

※ Schematic view

Solid support
(PCL)

I/O electrode
(Mg/PLCL-PLGA)

※ Layer structure

I/O pad

Pre-programmed shape
(by SMP programming)

PLCL/PLGA (~ 40 µm)

PCL

Mg or Mo (~ 500 nm)

Contact

PLCL/PLGA (~ 40 µm)

Fig. 8

**Fig. 9**

**Fig. 10**

## Fig. 11

## Fig. 12

**Fig. 13**

**Fig. 14**

**Fig. 15**

Fig. 16

**Fig. 17**

$$\varepsilon(y) = \Delta L/L = \frac{2\,\beta\,(R+y) - 2\,\beta\,(R)}{2\,\beta\,R} = y/R$$

Distance from N.P ↑ ⇒ applied strain ↑

Fig. 18

**Fig. 19**

**Fig. 20**

**Fig. 21**

**Fig. 22**

1. Device fabrication on temporal substrate    2. Protecting layer coating    3. Peel-off the device

Device (Mg, Mo, Si, SiO₂)
PI
Substrate (Si)

PBAT

Water soluble tape (WST)

4. Removal of PI    5. Attach to bottom layer    6. Removal of WST

RIE

7. SMP (top) coating    8. Substrate patterning    9. Detach from Si substrate

PLCL/PLGA

UV laser

# Fig. 23

1. SMP (bottom) coating

2. Substrate patterning

3. Attach center structure on substrate

PLCL/PLGA

PCL

4. Align single chassis device on substrate

5. Vertical fixation

6. Detach from Si substrate

# Fig. 24

Fig. 25

Fig. 26

**Fig. 27**

## INTERNATIONAL SEARCH REPORT

| International application No. |
| --- |
| **PCT/KR2024/000733** |

**A.    CLASSIFICATION OF SUBJECT MATTER**

**A61B 5/293**(2021.01)i; **A61B 5/263**(2021.01)i; **A61B 5/257**(2021.01)i; **A61B 5/00**(2006.01)i; **A61B 17/00**(2006.01)i; **A61B 90/00**(2016.01)i; **A61M 5/142**(2006.01)i; **A61M 31/00**(2006.01)i; **A61N 1/36**(2006.01)i

According to International Patent Classification (IPC) or to both national classification and IPC

**B.    FIELDS SEARCHED**

Minimum documentation searched (classification system followed by classification symbols)

A61B 5/293(2021.01); A61B 5/00(2006.01); A61B 90/00(2016.01); A61M 29/00(2006.01); A61M 29/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 뇌(brain), 침습(invasive), 주입(insert), 방사형(radial shape), 가지부(branch)

**C.    DOCUMENTS CONSIDERED TO BE RELEVANT**

| Category* | Citation of document, with indication, where appropriate, of the relevant passages | Relevant to claim No. |
| --- | --- | --- |
| X | KR 10-2021-0113957 A (SEOUL NATIONAL UNIVERSITY R&DB FOUNDATION) 17 September 2021 (2021-09-17)<br>See paragraphs [0004]-[0116]; and claim 12. | 20-22,26 |
| Y | | 1-19,23-25,27,28 |
| Y | KR 10-2018-0043028 A (RESEARCH & BUSINESS FOUNDATION SUNGKYUNKWAN UNIVERSITY) 27 April 2018 (2018-04-27)<br>See paragraph [0025]. | 1-19,23-25,27,28 |
| Y | KR 10-2017-0106767 A (LEE, Il Kwon) 22 September 2017 (2017-09-22)<br>See paragraphs [0026], [0027] and [0030]. | 17-19 |
| A | JP 2004-519307 A (TAEWOONG MEDICAL CO., LTD. et al.) 02 July 2004 (2004-07-02)<br>See entire document. | 1-28 |

☑ Further documents are listed in the continuation of Box C.     ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **16 April 2024** | **17 April 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

International application No.

**PCT/KR2024/000733**

**C.    DOCUMENTS CONSIDERED TO BE RELEVANT**

| Category* | Citation of document, with indication, where appropriate, of the relevant passages | Relevant to claim No. |
|---|---|---|
| A | US 8152831 B2 (MAGNUSON et al.) 10 April 2012 (2012-04-10)<br>See entire document. | 1-28 |

Form PCT/ISA/210 (second sheet) (July 2022)

## INTERNATIONAL SEARCH REPORT

International application No.

**PCT/KR2024/000733**

| **Box No. II** | **Observations where certain claims were found unsearchable (Continuation of item 2 of first sheet)** |

This international search report has not been established in respect of certain claims under Article 17(2)(a) for the following reasons:

1. ☑   Claims Nos.: **29-32**
because they relate to subject matter not required to be searched by this Authority, namely:

Claims 29-32 pertain to a method for treatment of the human body by surgery, and thus pertain to a subject matter on which the International Searching Authority is not required to carry out an international search under the provisions of PCT Article 17(2)(a)(i) and PCT Rule 39.1(iv).

2. ☐   Claims Nos.:
because they relate to parts of the international application that do not comply with the prescribed requirements to such an extent that no meaningful international search can be carried out, specifically:

3. ☐   Claims Nos.:
because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

International application No.

**PCT/KR2024/000733**

| Patent document cited in search report | | | Publication date (day/month/year) | Patent family member(s) | | | Publication date (day/month/year) |
|---|---|---|---|---|---|---|---|
| KR | 10-2021-0113957 | A | 17 September 2021 | KR | 10-2535611 | B1 | 26 May 2023 |
| KR | 10-2018-0043028 | A | 27 April 2018 | KR | 10-1864033 | B1 | 05 July 2018 |
| KR | 10-2017-0106767 | A | 22 September 2017 | None | | | |
| JP | 2004-519307 | A | 02 July 2004 | CN | 100270790 | C | 23 August 2006 |
| | | | | CN | 100431920 | A | 23 July 2003 |
| | | | | EP | 1296739 | A1 | 02 April 2003 |
| | | | | EP | 1296739 | A4 | 30 July 2008 |
| | | | | JP | 3708923 | B2 | 19 October 2005 |
| | | | | KR | 10-0457630 | B1 | 18 November 2004 |
| | | | | KR | 10-2002-0078127 | A | 18 October 2002 |
| | | | | US | 2002-0147489 | A1 | 10 October 2002 |
| | | | | US | 2005-0209708 | A1 | 22 September 2005 |
| | | | | US | 6974472 | B2 | 13 December 2005 |
| | | | | WO | 02-081019 | A1 | 17 October 2002 |
| US | 8152831 | B2 | 10 April 2012 | US | 2007-0118173 | A1 | 24 May 2007 |
| | | | | WO | 2007-059349 | A1 | 24 May 2007 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101864033

- KR 101744395